# EUROPEAN PATENT SPECIFICATION

(11) **EP 2 145 590 B1**
(45) Date of publication and mention of the grant of the patent: **16.05.2012**
(21) Application number: 08425490.3
(22) Date of filing: 18.07.2008
(51) Int. Cl.: A61B 17/15, A61B 17/16

(54) **Kit for implanting modular knee prostheses**
Kit zur Implantation von modularen Knieprothesen
Kit pour l'implantation de prothèses de genou modulaires

(43) Date of publication of application: 20.01.2010
(73) Proprietor: Societa'Azionaria Materiale Ospedaliero - S.A.M.O. S.p.A., Frazione Cadriano, Bologna (IT)
(72) Inventor: Vandini, Lorenzo, 40017 San Giovanni in Persiceto (Prov. of Bologna) (IT)
(74) Representative: Modiano, Micaela Nadia

(56) References cited:
- EP-A- 0 947 169
- US-A- 6 077 270
- US-A1- 2003 114 859
- US-A1- 2004 267 266

## Description

The present invention relates to a kit for implanting modular knee prostheses, particularly for implanting secondary prostheses.

Prostheses (particularly knee prostheses) can be distinguished into primary and secondary depending on whether they constitute the first implantation of a prosthesis in a natural joint that has suffered damage or a joint in which a prosthesis is already inserted and has to be replaced with another one due to problems of various kinds that can arise.

Modular knee prostheses, used in the correction of primary knee prostheses, are provided with stems (rods of various materials used to insert them in the medullary cavity of a long bone) to fix the device to the diaphyseal canals (the term "diaphyseal" being used to reference the central part of long bones) of the femur and tibia.

Known surgical techniques provide for preparation of the diaphyseal canals with a series of reamers, followed by the use of suitable cutting guides, which are keyed to the last reamer left in place, in order to level the surfaces of the bone epiphyses and adapt them to the new implant.

Finally, test components which in practice imitate the shape and characteristics of the ones that will be subsequently implanted (elements generally known as simulacra) are used to check the correctness or not of the articular function.

First of all, it has to be pointed out that the described technique provides for a considerable quantity of auxiliary components (i.e., of additional and preparatory components); this quantity of components entails difficulties in management on the part of health workers (storage, transport, sterilization) and most of all increases the complexity of operating procedures during surgery.

During a corrective operation, the anatomical references on the basis of which the primary prosthetic components are positioned are not present (since during the surgery to implant the primary prosthesis they were eliminated or damaged as a consequence of abrasive action during use of the already-implanted primary prosthesis), and therefore other less defined and reliable reference points are used as reference.

The bone ends are prepared independently of each other (the femur independently of the tibia), and articular functionality is then verified by applying to the prepared bone ends simulacra of the implantable components. If corrections are required, which is probable in this situation, it is necessary to remove the simulacra and reposition the reamers, guides, et cetera.

US-6 077 270 discloses a method and apparatus for locating bone cuts at the distal condylar femur region to receive a femoral prosthesis and to coordinate tibial and patellar resection and replacement with femoral resection and replacement as defined in the preamble of claim 1.

US 2004/267266 discloses a modular tapered reamer for bone preparation and associated method.

The aim of the present invention is to provide a kit for implanting modular knee prostheses which is constituted by a minimum number of mutually cooperating components.

Within this aim, an object of the present invention is to provide a kit for implanting modular knee prostheses that is adapted to be arranged on the respective bone surfaces precisely, utilising fixed reference points that are simple to locate.

Another object of the present invention is to provide a kit for implanting modular knee prostheses that allows corrections and adjustments regarding the position preset for the prosthesis.

Another object of the present invention is to provide a kit for implanting modular knee prostheses that has a low cost, is relatively simple to provide in practice and safe in application.

This aim and these and other objects, (which will become better apparent hereinafter, are achieved by the present kit for implanting modular knee prostheses, as defined in claim 1.

Further characteristics and advantages of the invention will become better apparent and evident from the following detailed description of a preferred but not exclusive embodiment of a kit for implanting modular knee prostheses, illustrated by way of non-limiting example in the accompanying drawings, wherein:

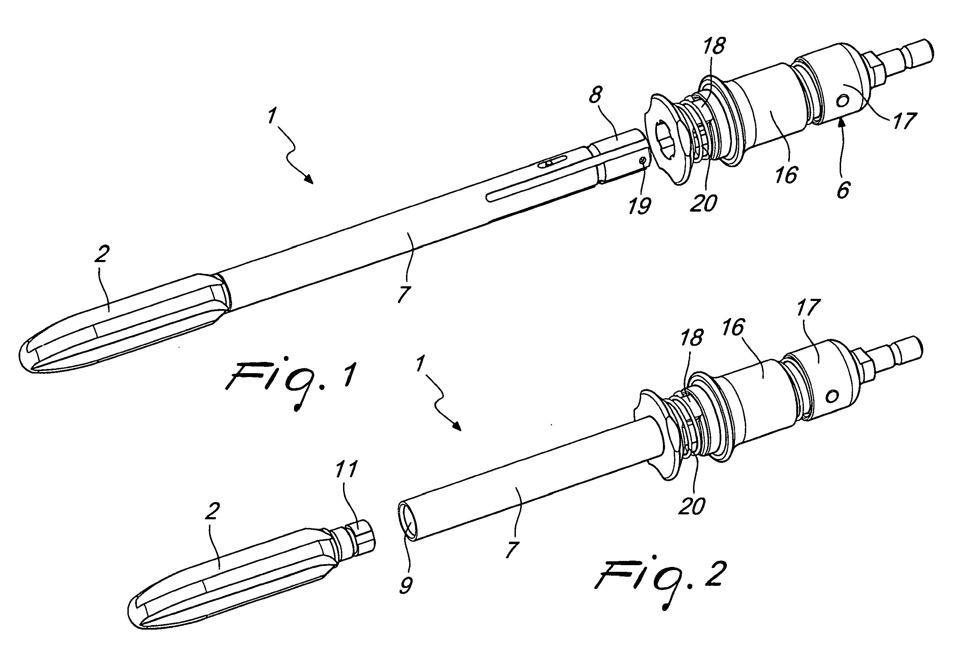

Figure 1 is a perspective view of a modular reaming assembly of a kit for implanting modular knee prostheses according to the invention;
Figure 2 is a perspective view of a modular reaming assembly, provided with a tubular body of limited length, of a kit for implanting modular knee prostheses according to the invention;

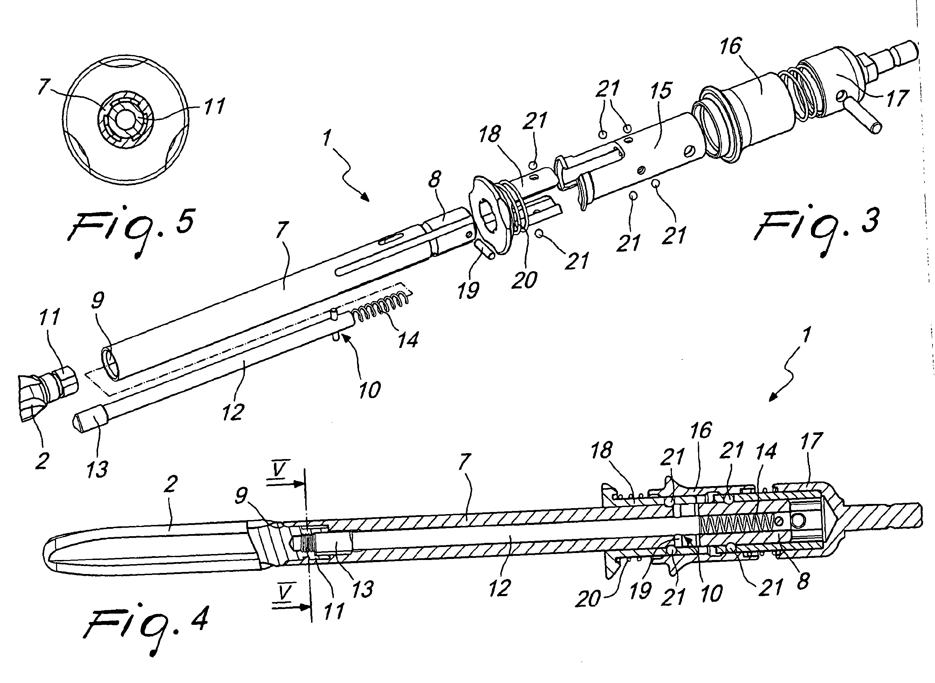

Figure 3 is an exploded perspective view of a modular reaming assembly of a kit for implanting modular knee prostheses according to the invention;
Figure 4 is a sectional side view, taken along an axial longitudinal plane, of a modular reaming assembly of a kit for implanting modular knee prostheses according to the invention;
Figure 5 is a sectional view, whose line V-V is shown in Figure 4;

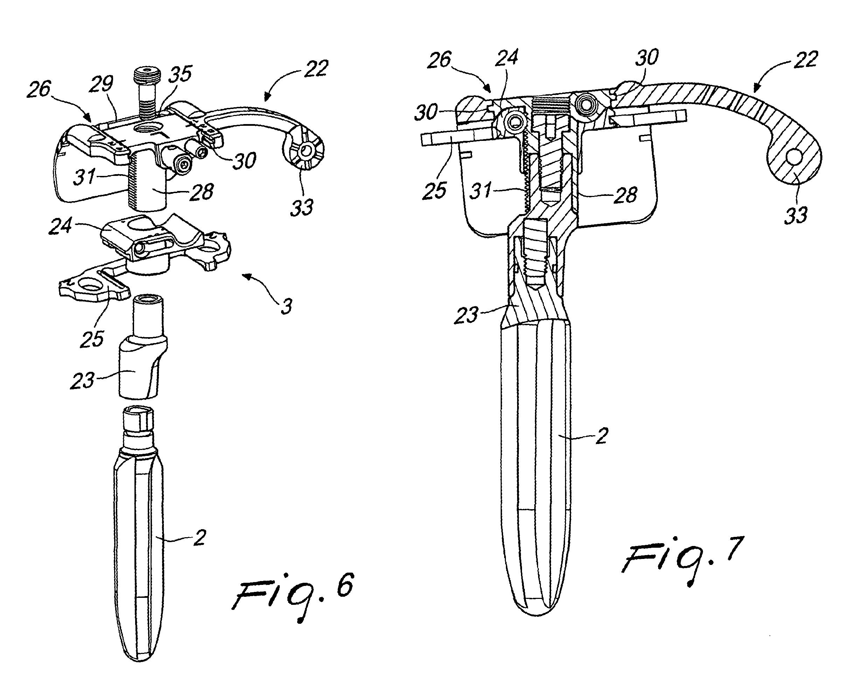

Figure 6 is an exploded perspective view of an adjustable unit associated with a cutting tool of a kit for implanting modular knee prostheses according to the invention;
Figure 7 is a sectional side view, taken along an axial longitudinal plane, of an adjustable unit which is associated with a cutting tool of a kit for implanting modular knee prostheses according to the invention;

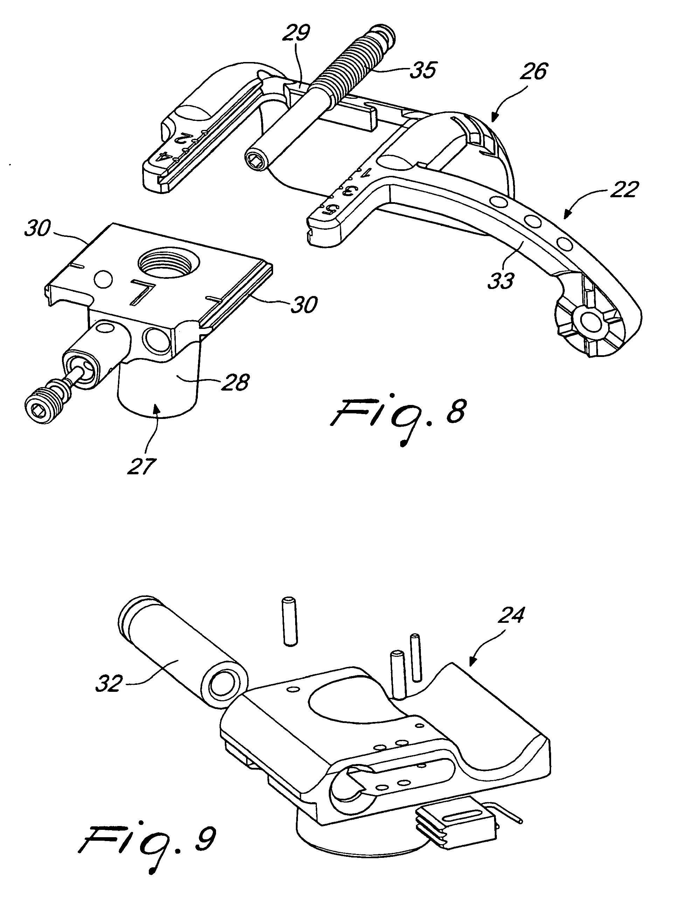

Figure 8 is an exploded perspective view of a supporting structure of a kit for implanting modular knee prostheses according to the invention;
Figure 9 is an exploded perspective view of a contoured collar of a kit for implanting modular knee prostheses according to the invention;

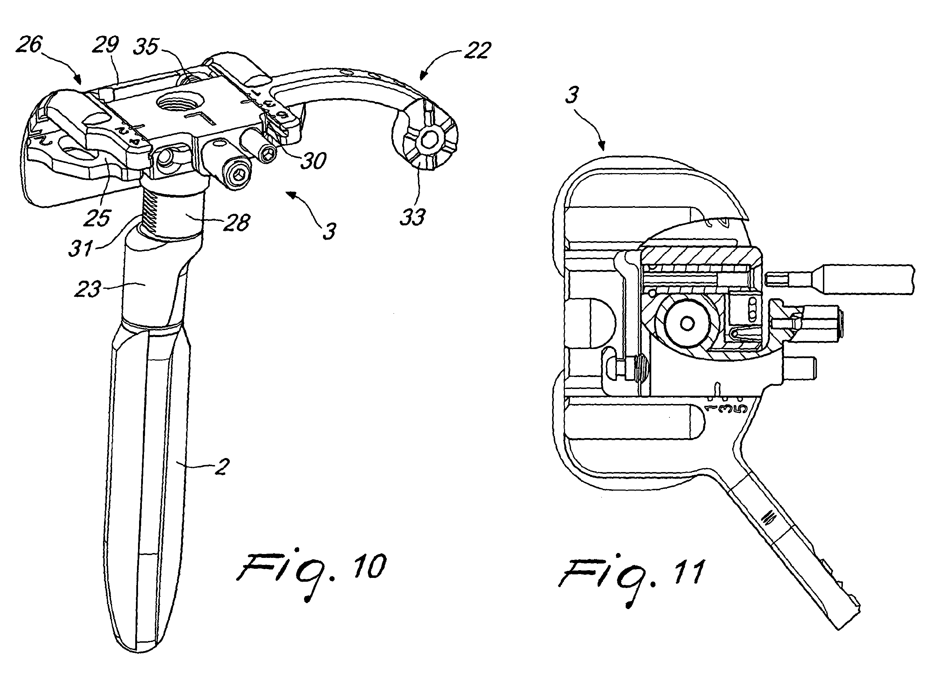

Figure 10 is a perspective view of an adjustable unit associated with a cutting tool of a kit for implanting modular knee prostheses according to the invention;
Figure 11 is a sectional front view, taken along a transverse plane, of an adjustable unit of a kit for implanting modular knee prostheses according to the invention;

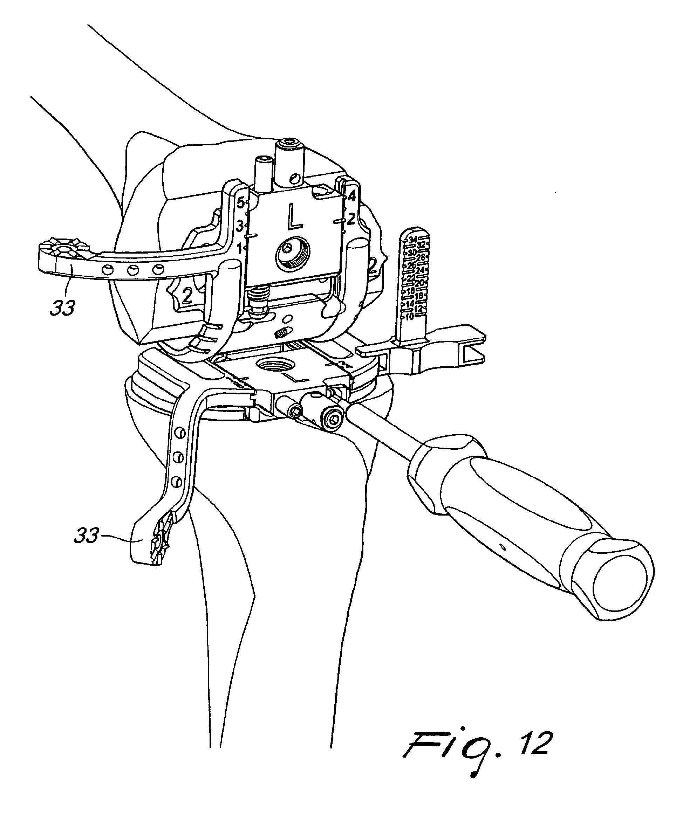

Figure 12 is a perspective view of a kit for implanting modular knee prostheses according to the invention in a first step of use;
Figure 13 is a perspective view of a kit for implanting modular knee prostheses according to the invention in a second step of use;

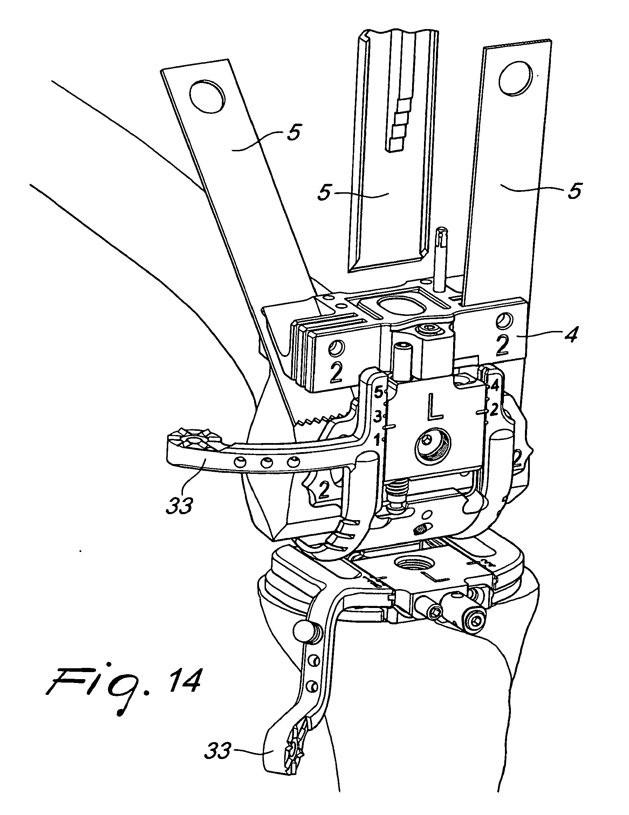

Figure 14 is a perspective view of a kit for implanting modular knee prostheses according to the invention in a third step of use;

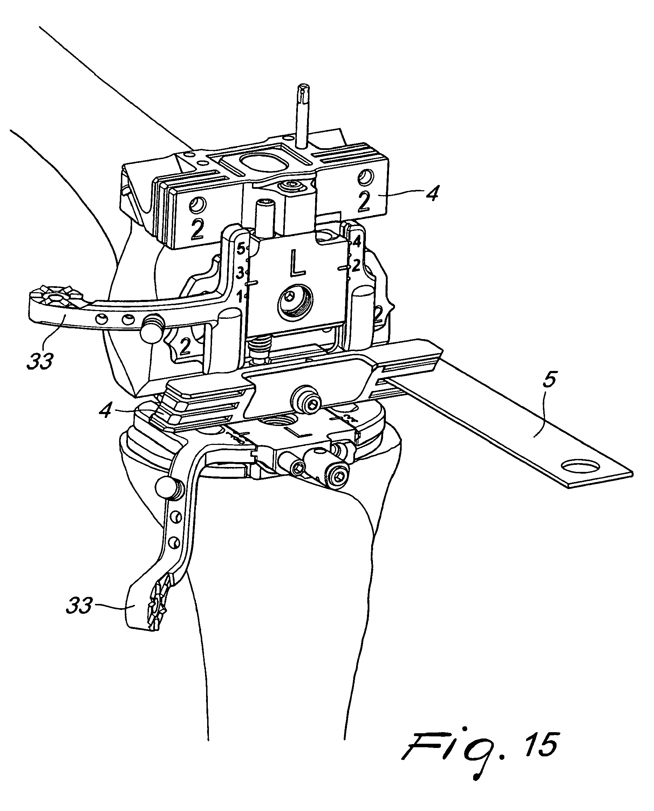

Figure 15 is a perspective view of a kit for implanting modular knee prostheses according to the invention in a fourth step of use;

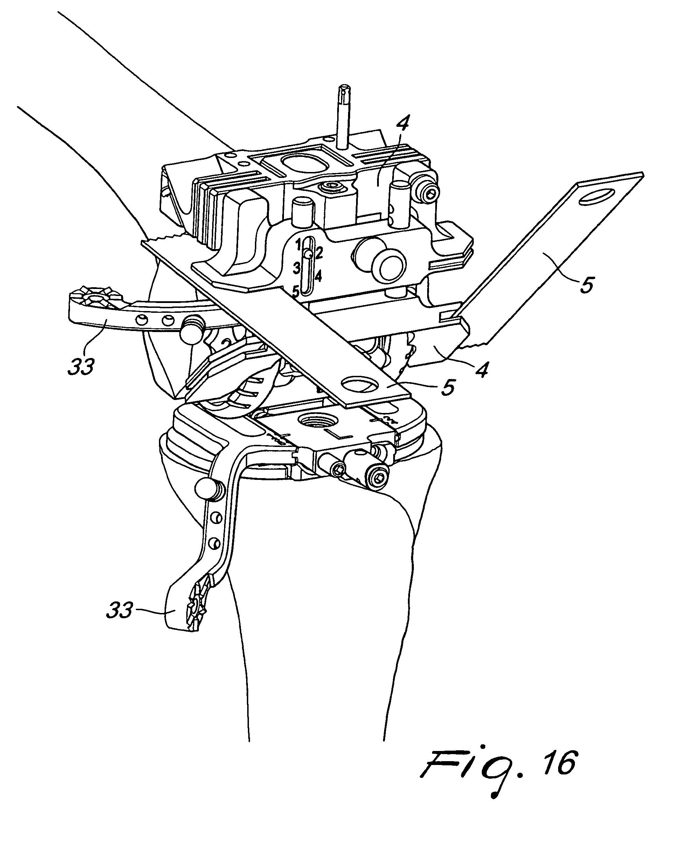

Figure 16 is a perspective view of a kit for implanting modular knee prostheses according to the invention in a fifth step of use;

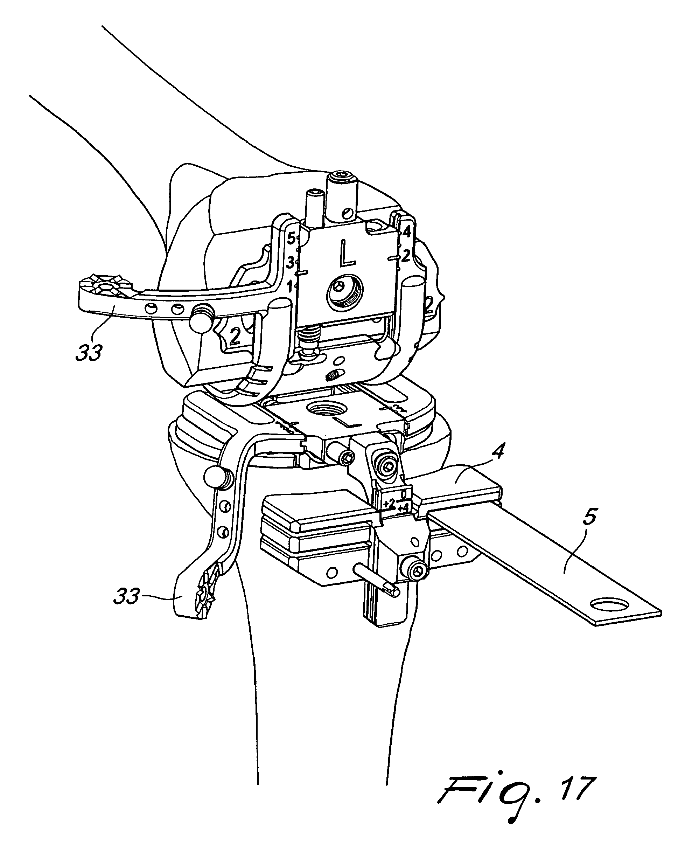

Figure 17 is a perspective view of a kit for implanting modular knee prostheses according to the invention in a sixth step of use;

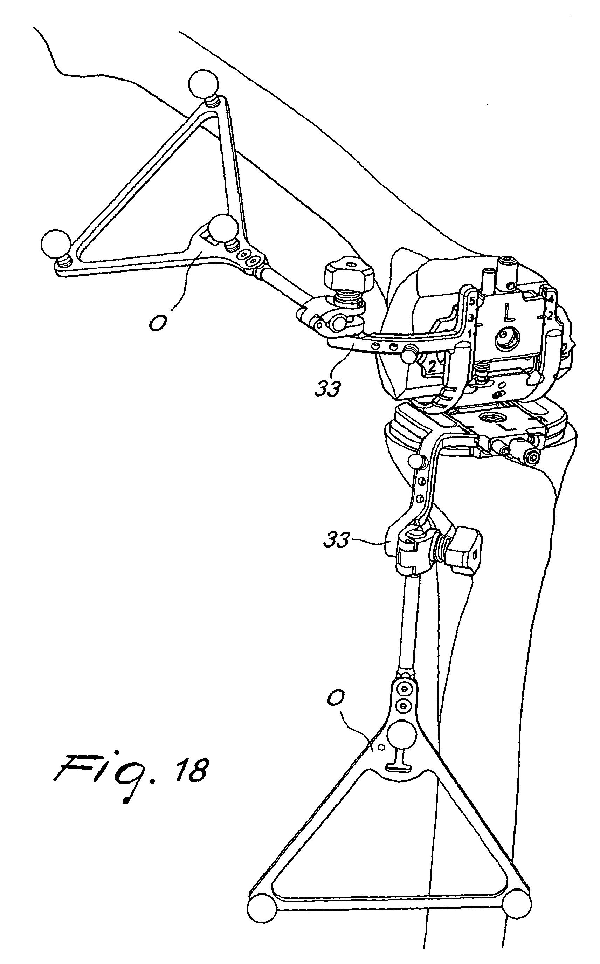

Figure 18 is a perspective view of a kit for implanting modular knee prostheses according to the invention in a seventh step of use.

With reference to the figures, a kit for implanting modular knee prostheses is illustrated which comprises a modular reaming assembly 1, which is provided with a terminal cutting tool 2 of the removable type.

The cutting tool 2 is in practice an actual reamer for surgical use: a reamer is a manual tool that allows to precision-finish the surgical site of implantation in order to achieve the maximum geometric match with the prosthetic stem that will be accommodated therein; substantially, it is used in case of dense cortical bone.

The kit further comprises an adjustable unit 3 for defining the installation position of the prosthesis and guiding templates 4, which can be associated (generally detachably) with the unit 3, for suitable cutting elements 5 which are necessary to cut bone portions that are incompatible with the prosthesis to be implanted.

The modular reaming assembly 1 comprises a handle 6 (which in practice may also not act as an actual handle but may act as a joint/device for regulated transmission of motion).

The handle 6 can be associated with a respective movement apparatus (not shown in the figure).

At least one tubular body 7 is associated, by means of one of its ends 8, with the handle 6 and by means of an opposite end 9 with the cutting tool 2. Each tubular body 7 has its own length, in order to define a set of bodies 7 which are mutually interchangeable in order to be able to choose the one whose length is best suited for the particular type of surgical procedure (as a function of the requirements of the medical staff and of the dimensional parameters of the patient).

The tubular body 7 accommodates internally suitable transmission means 10 for transferring motion from a transmission shaft of the handle 6 to a portion 11 for fixing the cutting tool 2.

Simplifying the structure of the modular assembly 1, it can be noted that it is constituted by the tubular body 7, inside which a bolt 12 (which belongs to the transmission means 10) slides; an end 13 of the bolt 12 on the side of the tool 2 is not axially symmetrical. The axial position of the bolt 12 is contrasted by a spring 14, which keeps it pressed toward the cutting tool 2. The cutting tool 2 is provided with a cylindrical coupling (the fixing portion 11).

Longitudinal and circumferential slots are provided externally and the seat for the bolt 12 and a threaded hole are provided internally.

On the inner face of the tubular body 7, on the tool side, there are notches that enter the longitudinal slots of the tool coupling 2. By turning the cutting tool 2, the notches enter the circumferential slot, locking its axial sliding. The rotation of the cutting tool 2 is blocked by the insertion of the bolt 12 in the seat formed on the cutting tool 2.

The so-called handle 6 is composed of a tubular core 15 and two sleeves 16 and 18, which can slide thereon. The core 15 is jointly connected to a standardized interface shank 17, which allows connection to suitable movement carousels.

The first sleeve 18 allows the axial sliding of the bolt 12 by acting on a transverse pin 19 that passes through the wall of the tubular body 7 within slots, with the interposition of balls 21 that cross the wall of the sleeve 18 and partly protrude from it.

The core 15 is jointly connected to the tubular body 7 by means of a slotted longitudinal profile, which constrains rotation, and by means of the balls 21.

The second sleeve 16, which can slide on the outside of the core 15, if retracted, frees both the transverse pin 19 of the bolt 12 and the circumferential slot of the tubular body 7 from the axial constraint determined by the balls 21, allowing to extract the handle 6.

With the tubular body 7 accommodated within the handle 6, it is possible to fix and replace the cutting tool 2 on the body 7 by acting on the first sleeve 18. By extracting the handle 6 without disengaging the cutting tool 2 from the tubular body 7, such tubular body remains fastened on the tool 2, giving rise to an object that can be used as an ordinary reamer, which can be used in the context of a conventional surgical technique.

The cutting tool 2 can be screwed onto a test prosthetic component and act as a test stem.

The apparatus for moving the cutting tool 2 (apparatus to be connected to the handle 6) is an electric motor: the rotation imparted by it ensures the removal of material by the tool 2 during the reaming of the diaphyseal part of the bone.

As an alternative, the movement apparatus can be constituted by a grip frame (for example of the T-shaped type) for manual rotation of the cutting tool 2 during reaming of the diaphyseal part of the bone.

The adjustable unit 3 comprises a framework 22, whose orientation and dimensions are adjustable, and an interface coupling 23, which is optionally available in various dimensions and can be mated stably to the cutting tool 2.

The coupling 23 is provided with a head, which can be coupled rigidly to the framework 22 to fix it to the bone. The cutting tool 2 can also be stably fixed directly on the framework 22, without the interposition of any coupling 23.

The framework 22 comprises a profiled collar 24, a base 25 and a supporting structure 26.

The base 25, optionally available in various sizes, can be coupled detachably on the supporting structure 26.

The supporting structure 26 comprises a carousel 27, which is provided with a sleeve 28 that can be accommodated within the profiled collar 24, and a slider 29, which can slide on respective lateral tracks 30 of the carousel 27.

The mutual position of the carousel 27 and of the slider 29 can be modified by way of suitable adjustment means.

The sleeve 28 of the carousel 27 is provided with a set of teeth 31 along at least one region of its lateral surface.

In such region, once the carousel 27 has been fitted on the collar 24, the set of teeth 31 faces, and is proximate to, a suitable toothed element 32 (accommodated within the collar 24): the element 32 has a respective set of teeth, which is shaped complementarily to the set of teeth of such region, in order to adjust the mutual position of the carousel 27, and therefore also of the slider 29, with respect to the profiled collar 24 and therefore to the base 25.

Adjustment is obtained by moving the toothed element 32 while it is engaged on the set of teeth 31.

The slider 29 is moved with respect to the carousel 27 by an actuation screw 35. There is a graduated scale that indicates the correspondence between the position of the slider 29 and the corresponding prosthetic component size.

The guiding templates 4 are coupled detachably to suitable protrusions of the unit 3 and form suitable guiding paths for the cutting elements 5 for cutting bone portions that are incompatible with the prosthesis to be implanted.

The framework 22 comprises a protruding arm 33 for fixing a position detector, which is adapted to check the correct position of the components of the kit and therefore of the prosthesis subsequently installed according to the configuration determined by such kit.

The protruding arm 33 can be jointly connected to the supporting structure 26 (as shown in the figure) rather than to the profiled collar 24.

Advantageously, the unit 3 according to the invention constitutes an adjustable test component (formed by the cutting tool 2, by the adjustable unit 3 and by all the components that can be associated with them); the unit 3 assumes two shapes, a femoral one and a tibial one.

The units 3 are positioned on the bone ends (in practice they occupy the space left by the removed primary prosthetic components), allowing to modify the main dimensional parameters that control the development of the series of implantable components, i.e., size and thickness, and obtain iteratively an objective verification of articular function.

The unit 3 therefore defines evidently the level of the new joint; it is simpler, by means of a suitable measurement system (the figure illustrates a graduated accessory 34 and various areas of the various components have similar graduated measurement portions), to position the units 3 in relation to the anatomical references simply and intuitively.

The units 3 are further provided with arms 33, which support suitable optical, electromagnetic or mechanical position sensors, or if a surgical technique assisted by a navigation system is adopted.

The carousel 27 is connected to the cutting tool 2 by way of a fixing screw, with the optional interposition of the interface coupling 23, which introduces an offset between the bodies mentioned above or extends the length of the tool 2.

It should be noted that between the set of teeth 31 and the corresponding toothed element 32 there is a one-way toothed block, which is contrasted by a spring, which engages the toothed element 32 and prevents its rotation. In order to act on the toothed element 32, which has an axial hole whose cross-section is not axially symmetrical, a suitable key is used which is characterized by a conical portion that allows to displace the one-way block during insertion in the toothed element 32.

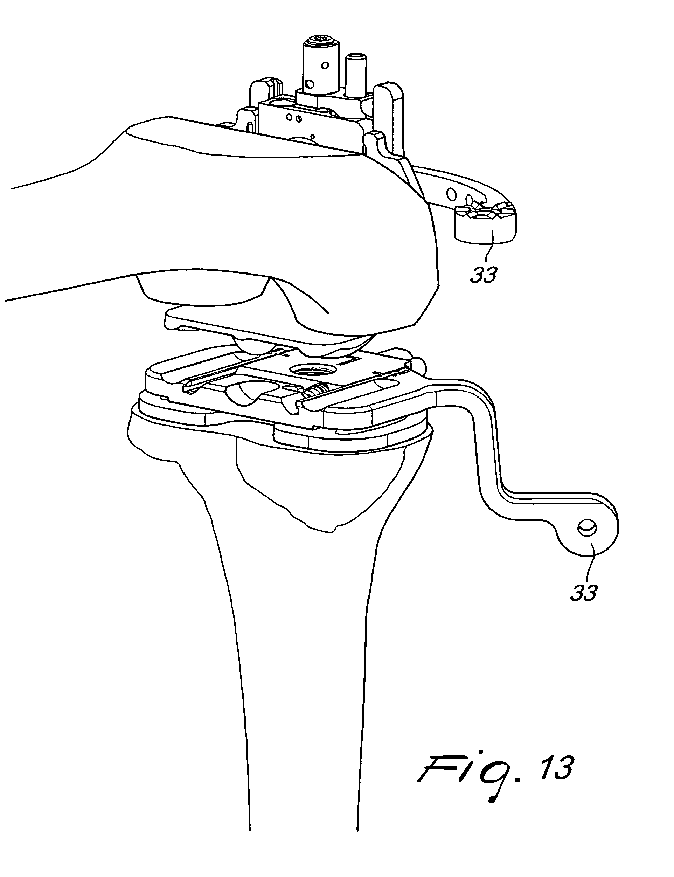

The slider 29 suitable for use on the femoral portion of the patient has a protrusion which, at approximately 90° of flexing of the joint, enters a cavity of the tibial slider 29, thus determining the relative anteroposterior position of femur and tibia, in a manner similar to what occurs with the implant (as shown in Figure 13).

The unit 3 for adjusting tibial configuration can lack the anteroposterior degree of freedom (slider 29 and carousel 27 provided monolithically).

The present kit can be used by following the method described hereafter.

The reamers (tools 2)/test stems, which will be the ones used to prepare the medullary canals and any interface couplings 23 (in order to provide offset or elongation), chosen during preparation of the canals on the units 3 are fitted. The bases 25 that best cover the bone ends are selected as a function of the characteristics of the patient and are fixed to the units 3.

The size of the prosthetic system is estimated, for example by measuring the removed components, and this size is set with the maneuvering screws 35. The distal proximal thickness of the units 3 is reduced to a minimum and the units 3 are placed on the bone ends and the joint is reduced.

By means of a ruler (graduated accessory 34) it is possible to estimate the distance between the tibial slider 29 (articular level) and the chosen anatomical references and, by using the suitable key, the appropriate adjustments are performed on the two degrees of femoral and tibial freedom until a trim that is satisfactory in terms of articular function is achieved.

Having done this, rotation about the test stems is fixed by inserting one or more pins in the provided holes (provided in the components that constitute the units 3 necessary indeed to fix the unit to the bone).

Then a template 4 is positioned on the femoral supporting structure 26 and allows to control and pick up distal femoral cuts and the anterior bevel.

An additional template 4, which can be expanded according to the chosen size and allows to control and pick up the anterior cut and rear bevel, can be fixed to the template 4.

An additional template 4, which allows to control and pick up the posterior cut, is arranged on the femoral supporting structure 26.

A guide is fixed on the tibial supporting structure 26 and a respective template 4 is lowered onto it from above and allows to control and pick up tibial cutting.

It has thus been shown that the invention achieves the proposed aim and objects.

The invention thus conceived is susceptible of numerous modifications and variations, all of which are within the scope of the appended claims.

All the details may further be replaced with other technically equivalent ones.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the shapes and dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A kit for implanting modular knee prostheses, comprising a modular reaming assembly (1), provided with a terminal cutting tool (2) of the removable type, an adjustable unit (3) for defining the installation position of the prosthesis and guiding templates (4), which can be associated with said unit (3), for cutting elements (5) required to cut bone portions that are incompatible with said prosthesis, said adjustable unit (3) comprising a framework (22) whose orientation and dimensions are adjustable and which can be coupled stably to the cutting tool (2), with the optional interposition of an interface coupling (23), when said cutting tool (2) is inserted within the bone, said framework (22) being fixable to said bone, said framework (22) comprising a profiled collar (24), a base (25) which can be fixed detachably to said collar (24), and a supporting structure (26), **characterized in that** said supporting structure (26) comprises a carousel (27), which is provided with a sleeve (28) which can be accommodated within said profiled collar (24), and a slider (29), which can slide on respective lateral tracks (30) of said carousel (27), the mutual position of said carousel (27) and of said slider (29) being modifiable by way of adjustment means.

2. The kit according to claim 1, wherein said modular reaming assembly (1) comprises a handle (6), which can be associated with a respective movement apparatus, at least one tubular body (7) which is associated by means of one of its ends (8) with said handle (6) and by means of the opposite end (9) with said terminal cutting tool (2), said tubular body (7) accommodating suitable transmission means (10) for transferring motion from a transmission shaft of said handle (6) to the fixing portion (11) of said cutting tool (2).

3. The kit according to claim 2, wherein said handle (6) comprises a seat which is provided with suitable slotted profiles for detachable coupling to the respective end (8), whose shape and dimensions are complementary to those of said seat, of said tubular body (7).

4. The kit according to claim 2, wherein said tubular body (7) comprises a receptacle (9), which is provided with suitable contoured profiles, for detachable coupling with the respective fixing portion (11), whose shape and dimensions are complementary to those of said receptacle (9), of said cutting tool (2).

5. The kit according to claim 2, wherein said movement apparatus is an electric motor for actuating the cutting tool (2) during the reaming of the diaphyseal part of the bone.

6. The kit according to claim 2, wherein said movement apparatus is a grip frame for manual rotation of the cutting tool (2) during the reaming of the diaphyseal part of the bone.

7. The kit according to claim 1, wherein the sleeve (28) of said carousel (27) is provided with a set of teeth (31) along at least one region of the lateral surface of the sleeve (28), when the carousel (27) is fitted on the collar (24) said set of teeth (31) facing and being proximate to a toothed element (32), whose set of teeth is complementary to the set of teeth of said region, in order to adjust the mutual position of said carousel (27), therefore also of said slider (29), with respect to said profiled collar (24) and said base (25), adjustment being obtained by moving said toothed element (32) while it is engaged on the set of teeth (31).

8. The kit according to claim 1, wherein said guiding templates (4) mate detachably with suitable protrusions of said unit (3), defining suitable guiding paths for said cutting elements (5) for cutting bone portions that are not compatible with said prosthesis.

9. The kit according to one or more of the preceding claims, wherein said supporting structure (26) comprises a protruding arm (33) for fixing a position detector adapted to check the correct position of the components of the kit and therefore of the prosthesis installed subsequently according to the configuration determined by said kit.

## Patentansprüche

1. Set zum Implantieren modularer Knieprothesen, das eine module Aufweiteeinheit (1), die mit einem am Ende angeordneten Schneidwerkzeug (2) des entfernbaren Typs ausgestattet ist, eine einstellbare Einheit (3) zum Definieren der Installationsposition der Prothese und Führungsschablonen (4), die mit der Einheit (3) verbunden werden können, für Schneidelemente (5) umfasst, die zum Schneiden von Knochenanteilen benötigt werden, die mit der Prothese unvereinbar sind, wobei die einstellbare Einheit (3) einen Rahmen (22) umfasst, dessen Orientierung und Ausdehnungen einstellbar sind und der stabil mit dem Schneidwerkzeug (2) mit der optionalen Zwischenschaltung einer Kopplungsschnittstelle (23) verbunden werden kann, wenn das Schneidwerkzeug (2) in den Knochen eingeführt ist, wobei der Rahmen (22) an dem Knochen befestigbar ist und wobei der Rahmen (22) einen profilierten Kragen (24), eine Basis (25), die mit dem Kragen (24) lösbar verbunden werden kann, und eine Stützstruktur (26) umfasst, **dadurch gekennzeichnet, dass** die Stützstruktur (26) eine Schaftaufnahme (27), die mit einer Hülse (28) versehen ist, die innerhalb des profilierten Kragens (24) aufgenommen werden kann, und einen Gleiter (29) umfasst, der entlang von entsprechenden seitlichen Schienen (30) der Schaftaufnahme (27) gleiten kann, wobei die wechselseitigen Positionen der Schaftaufnahme (27) und des Gleiters (29) mittels Einstellmitteln veränderbar sind.

2. Set nach Anspruch 1, wobei die modulare Aufweiteeinheit (1) ein Griffstück (6), das mit einem entsprechenden Bewegungsapparat verbunden werden kann und wenigstens einen rohrförmigen Körper (7) umfasst, der mit einem seiner Enden (8) mit dem Griffstück (6) und mit dem gegenüberliegenden Ende (9) mit dem am Ende angeordneten Schneidwerkzeug (2) verbunden ist, wobei der rohrförmige Körper (7) geeignete Übertragungsmittel (10) zum überragen von Bewegung von einer Übertragungswelle des Griffstückes (6) zu dem Befestigungsanteil (11) des Schneidwerkzeuges (2) beinhaltet.

3. Set nach Anspruch 2, wobei das Griffstück (6) einen Sitz umfasst, der mit geeigneten geschlitzten Profilen versehen ist zum lösbaren Verbinden mit dem entsprechenden Ende (8), dessen Form und Ausdehnungen komplementär zu denen des Sitzes sind, des rohrförmigen Körpers (7).

4. Set nach Anspruch 2, wobei der rohrförmige Körper (7) eine Aufnahme (9) umfasst, die mit geeigneten geformten Profilen versehen ist zum lösbaren Verbinden mit dem entsprechenden Befestigungsanteil (11), dessen Form und Ausdehnungen komplementär zu denen der Aufnahme (9) sind, des Schneidwerkzeuges (2).

5. Set nach Anspruch 2, wobei der Bewegungsapparat ein elektrischer Motor zum Betreiben des Schneidwerkzeuges (2) während des Aufweitens des diaphysären Teils des Knochens ist.

6. Set nach Anspruch 2, wobei der Bewegungsapparat einen Griffrahmen zur manuellen Drehung des Schneidwerkzeuges (2) während des Aufweitens des diaphysären Teils des Knochens ist.

7. Set nach Anspruch 1, wobei die Hülse (28) der Schaftaufnahme (27) mit einem Satz von Zähnen (31) entlang wenigstens eines Bereichs der seitlichen Oberfläche der Hülse (28) ausgestattet ist, wobei, wenn die Schaftaufnahme (27) an dem Kragen (24) montiert ist, der Satz von Zähnen (31) auf ein gezahntes Element (32) zeigt und zu diesem benachbart ist, dessen Satz von Zähnen komplementär zu dem Satz von Zähnen des Bereichs ist, um die relative Position der Schaftaufnahme (27) und daher also auch des Gleiters (29), relativ zu dem profilierten Kragen (24) und der Basis (25) einzustellen, wobei die Einstellung durch Bewegen des gezahnten Elementes (32) erreicht wird, während es mit dem Satz von Zähnen (31) in Eingriff steht.

8. Set nach Anspruch 1, wobei die Führungsschablonen (4) lösbar mit geeigneten Vorsprüngen der Einheit (3) zusammenpassen, die geeignete Führungspfade für die Schneidelemente (5) zum Schneiden von Knochenanteilen definieren, die mit der Prothese unvereinbar sind.

9. Set nach einem oder mehreren der vorstehenden Ansprüche, wobei die Stützstruktur (26) einen herausstehenden Arm (33) zum Befestigen eines Positionsdektektors umfasst, der angepasst ist, um die richtige Position der Komponenten des Sets und daher der nachfolgend der durch das Set ermittelten Konfiguration entsprechend installierten Prothese zu überprüfen.

## Revendications

1. Kit pour implanter des prothèses de genou modulaires, comprenant un ensemble aléseur modulaire (1), prévu avec un outil de coupe terminal (2) du type amovible, une unité ajustable (3) pour définir la position d'installation de la prothèse et des gabarits de guidage (4) qui peuvent être associés à ladite unité (3) pour couper des éléments (5) nécessaires pour couper des parties osseuses qui sont incompatibles avec ladite prothèse, ladite unité ajustable (3) comprenant un châssis (22) dont l'orientation et les dimensions sont ajustables et qui peut être couplé de manière stable à l'outil de coupe (2), avec l'interposition facultative d'un couplage d'interface (23), lorsque ledit outil de coupe (2) est inséré à l'intérieur de l'os, ledit châssis (22) pouvant être fixé sur ledit os, ledit châssis (22) comprenant un collier profilé (24), une base (25) qui peut être fixée de manière détachable sur ledit collier (24), et une structure de support (26), **caractérisé en ce que** ladite structure de support (26) comprend un carrousel (27) comporte un manchon (28) qui peut être logé à l'intérieur dudit collier profilé (24), et une glissière (29) qui peut glisser sur des rails latéraux (30) respectifs dudit carrousel (27), la position mutuelle dudit carrousel (27) et de ladite glissière (29) pouvant être modifiée au moyen de moyens d'ajustement.

2. Kit selon la revendication 1, dans lequel ledit ensemble aléseur modulaire (1) comprend une poignée (6) qui peut être associée à un appareil de mouvement respectif, au moins un corps tubulaire (7) qui est associé au moyen de l'une de ses extrémités (8) à ladite poignée (6) et au moyen de l'extrémité opposée (9) audit outil de coupe terminal (2), ledit corps tubulaire (7) logeant des moyens de transmission (10) appropriés pour transférer le mouvement d'une tige de transmission de ladite poignée (6) à ladite partie de fixation (11) dudit outil de coupe (2).

3. Kit selon la revendication 2, dans lequel ladite poignée (6) comprend un siège comportant des profils fendus appropriés pour se coupler de manière détachable à l'extrémité (8) respective, dont la forme et les dimensions sont complémentaires de celles dudit siège dudit corps tubulaire (7).

4. Kit selon la revendication 2, dans lequel ledit corps tubulaire (7) comprend un réceptacle (9) qui comporte avec des profils façonnés appropriés, pour se coupler de manière détachable avec la partie de fixation (11) respective dont la forme et les dimensions sont complémentaires de celles dudit réceptacle (9) dudit outil de coupe (2).

5. Kit selon la revendication 2, dans lequel ledit appareil de mouvement est un moteur électrique pour actionner ledit outil de coupe (2) pendant l'alésage de la partie diaphysaire de l'os.

6. Kit selon la revendication 2, dans lequel ledit appareil de mouvement est un châssis de poignée pour la rotation manuelle de l'outil de coupe (2) pendant l'alésage de la partie diaphysaire de l'os.

7. Kit selon la revendication 1, dans lequel le manchon (28) dudit carrousel (27) comporte un ensemble de dents (31) le long d'au moins une région de la surface latérale du manchon (28), lorsque le carrousel (27) est monté sur le collier (24), ledit ensemble de dents (31) faisant face et étant à proximité d'un élément denté (32), dont l'ensemble de dents est complémentaire à l'ensemble de dents de ladite région, afin d'ajuster la position mutuelle dudit carrousel (27), par conséquent également de ladite glissière (29), par rapport audit collier profilé (24) et ladite base (25), l'ajustement étant obtenu en déplaçant ledit élément denté (32) alors qu'il est mis en prise sur l'ensemble de dents (31).

8. Kit selon la revendication 1, dans lequel lesdits gabarits de guidage (4) se couplent de manière détachable avec des saillies appropriées de ladite unité (3), définissant des trajectoires de guidage appropriées pour lesdits éléments de coupe (5) pour couper les parties osseuses qui ne sont pas compatibles avec ladite prothèse.

9. Kit selon une ou plusieurs des revendications précédentes, dans lequel ladite structure de support (26) comprend un bras en saillie (33) pour fixer un détecteur de position adapté pour vérifier la position correcte des composants du kit et par conséquent de la prothèse installée successivement selon la configuration déterminée par ledit kit.
